(19) Europäisches Patentamt
European Patent Office
Office européen des brevets

(11) **EP 4 393 318 A1**

(12) **EUROPEAN PATENT APPLICATION**
published in accordance with Art. 153(4) EPC

(43) Date of publication:
**03.07.2024 Bulletin 2024/27**

(21) Application number: **22861398.0**

(22) Date of filing: **24.08.2022**

(51) International Patent Classification (IPC):
**A23L 5/00** (2016.01)          **A23J 3/14** (2006.01)
**A23L 11/50** (2021.01)          **C12N 9/04** (2006.01)

(52) Cooperative Patent Classification (CPC):
**A23J 3/14; A23L 5/00; A23L 11/50; C12N 9/0004**

(86) International application number:
**PCT/JP2022/031885**

(87) International publication number:
**WO 2023/027110 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.08.2021 JP 2021136568**

(71) Applicant: **Amano Enzyme Inc.**
**Nagoya-shi**
**Aichi 460-8630 (JP)**

(72) Inventor: **SAKAI, Kiyota**
**Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

(54) **METHOD FOR PRODUCING VEGETABLE-PROTEIN-FERMENTED FOOD OR BEVERAGE**

(57) The object of the present invention is to provide a manufacturing technique with which it is possible to improve various properties (particularly properties such as stress, water retention properties, syneresis-suppressing properties, or digestive properties of a fermented food or beverage itself, or properties for shortening the fermentation time during production of a fermented food or beverage) relating to a fermented food or beverage using a plant-protein ingredient. In the production of a fermented food or beverage using a plant-protein ingredient, a plant-cell-wall-derived polysaccharide and a multicopper oxidase are caused to act, thereby improving various properties relating to the fermented food or beverage.

EP 4 393 318 A1

**Description**

TECHNICAL FIELD

The present invention relates to a method for producing plant-protein-fermented food or beverage. More specifically, the present invention relates to a technique for modifying characteristics such as stress, water retention properties, syneresis-suppressing properties, or digestive properties of a plant-protein-fermented food or beverage, or characteristics affecting the fermentation time during production thereof.

BACKGROUND ART

In recent years, in the food and beverage market, food or beverage containing a plant protein as a raw material has increased the share as an alternative to animal food and drink. Foods and beverages made from plant proteins were used to be targeted at a select group of consumers, such as vegetarians and vegans, but demand trends have changed in recent years. Due to the growing awareness of health consciousness, diet, environmental issues, animal welfare, etc., it is receiving renewed attention.

On the other hand, in the food and beverage market, fermented foods, which have been part of foods produced by traditional food preservation techniques, are being reconsidered as superfoods and are once again in the spotlight.

Along with these trends, there have also been examples in which fermented foods or beverages using plant-protein ingredients are produced. However, there has been little study on modification of such fermented food or beverage. Modification techniques that have been studied so far for a fermented food or beverage using a plant-protein ingredient is limited to the following. For example, Patent Document 1 discloses a method for producing bean curd and yogurt using a protein material crosslinked using laccase. Patent Document 2 discloses a method for producing soy milk hard yogurt by treating black soybeans with a plant tissue disintegrating/fibrinolytic enzyme and adding transglutaminase to the obtained whole soy milk.

The following techniques are known as techniques for modifying animal fermented food or beverage. For example, Patent Document 3 discloses a method for producing yogurt having the original smooth texture inherent to yoghurt without causing syneresis by adding transglutaminase to milk raw materials. Patent Document 4 discloses that the addition of glucose oxidase in the production step of fermented milk significantly suppresses syneresis and an increase in the particle size of milk protein caused by the aggregation of milk proteins as compared with the case where glucose oxidase is not added. Patent Document 5 discloses a method for manufacturing fermented milk with a smooth texture and reduced whey separation by blending peroxidase and whey protein concentrate and/or whey protein isolate into a yogurt mix and fermenting it with lactic acid bacteria.

PRIOR ART DOCUMENT

PATENT DOCUMENTS

Patent Document 1: Japanese Patent Laid-open Publication No. H11-276162
Patent Document 2: Japanese Patent Laid-open Publication No. 2009-273412
Patent Document 3: Japanese Patent Laid-open Publication No. H06-197688
Patent Document 4: WO2012/121090
Patent Document 5: Japanese Patent Laid-open Publication No. H06-276933

SUMMARY OF THE INVENTION

PROBLEMS TO BE SOLVED BY THE INVENTION

A technique for modifying a fermented food or beverage using a plant-protein ingredient has hardly been studied, and the fermented food and beverage is still being in development. In order to further expand the share of the fermented food and beverage in the food and beverage market, it is desired to improve various properties that affect palatability or production efficiency (such as stress, water retention properties, syneresis-suppressing properties, or digestive properties of a fermented food and beverage itself, or properties that affect the fermentation time during production of the fermented food or beverage).

Then, it is an object of the present invention to provide a manufacturing technique with which it is possible to improve various properties (particularly properties such as stress, water retention properties, syneresis-suppressing properties, or digestive properties in a fermented food or beverage, or properties that affect the fermentation time during production of the fermented food or beverage) related to a fermented food or beverage using a plant-protein ingredient.

MEANS FOR SOLVING THE PROBLEM

The present inventors have found that when a combination of multicopper oxidase and plant-cell-wall-derived polysaccharide, which is a combination of enzymes that have not been previously used in any plant-based and animal-based fermented food or beverage as a means to improve the properties of such a food or beverage is applied to a process for producing the fermented food or beverage, various properties of the resulting plant-protein-fermented food or beverage can be improved. The present invention has been completed by further studies based on such findings.

That is, the present invention provides the inventions of the following aspects.

Item 1. A method for producing a plant-protein-fermented food or beverage, including the steps of: fermenting a plant-protein ingredient; and performing a treatment with a plant-cell-wall-derived polysaccharide and a multicopper oxidase.

Item 2. The method according to Item 1, in which the plant-cell-wall-derived polysaccharide is pectin.

Item 3. The method according to Item 2, in which the pectin is derived from beet.

Item 4. The method according to any one of Items 1 to 3, in which the multicopper oxidase is laccase.

Item 5. The method according to any one of Items 1 to 4, in which the plant-protein ingredient is plant milk.

Item 6. The method according to any one of Items 1 to 5, in which the plant-protein-fermented food or beverage is plant yogurt.

Item 7. A modifier for a plant-protein-fermented food or beverage, the modifier including a plant-cell-wall-derived polysaccharide and a multicopper oxidase.

Item 8. The modifier according to Item 7, used as a stress improver for a plant-protein-fermented food or beverage.

Item 9. The modifier according to Item 7, used as a water retention property improver for a plant-protein-fermented food or beverage.

Item 10. The modifier according to Item 7, used as a syneresis inhibitor for a plant-protein-fermented food or beverage.

Item 11. The modifier according to Item 7, used as a digestive property improver for a plant-protein-fermented food or beverage.

Item 12. An agent for shortening fermentation time in production of a plant-protein-fermented food or beverage, the agent including a plant-cell-wall-derived polysaccharide and a multicopper oxidase.

ADVANTAGES OF THE INVENTION

With the present invention, it is possible to improve various characteristics (particularly improve characteristics such as stress, water retention properties, syneresis-suppressing properties, or digestive properties in a fermented food or beverage itself, or shorten the fermentation time during production of the fermented food or beverage) related to a fermented food or beverage using a plant-protein ingredient.

BRIEF DESCRIPTION OF THE DRAWING

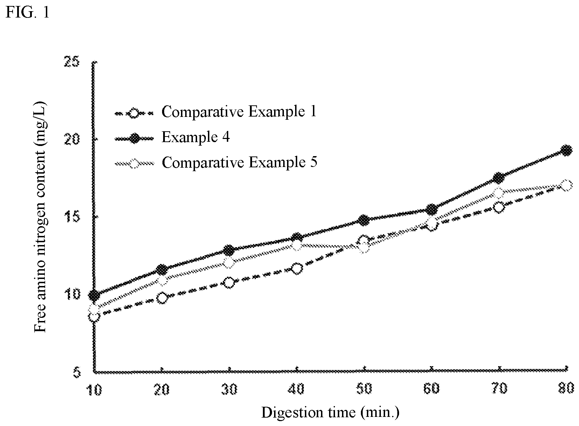

Fig. 1 shows effects of improving the digestive properties of soy milk yogurt obtained by the production method of the present invention, which was confirmed in Test Example 4.

EMBODIMENTS OF THE INVENTION

1. Method for producing plant-protein-fermented food or beverage

A method for producing a plant-protein-fermented food or beverage according to the present invention is characterized in including the steps of: fermenting a plant-protein ingredient; and performing a treatment with a plant-cell-wall-derived polysaccharide and a multicopper oxidase. This makes it possible to improve various properties related to the obtained plant-protein-fermented food or beverage. The method for producing plant-protein-fermented food or beverage according to the present invention is described below in detail.

In the following description, various properties related to the plant-protein-fermented food or beverage refer to at least any of the following: properties such as stress, water retention properties, syneresis-suppressing properties, and digestive properties that the fermented food or beverage itself owns; and properties that affect the fermentation time during a period in which the fermented food or beverage is produced. In the following description, improving various characteristics related to the plant-protein-fermented food or beverage refers to at least any of the following: improving characteristics such as stress, water retention properties, syneresis-suppressing properties, and digestive properties that the fermented food or beverage itself owns; and shortening the fermentation time during a period in which the fermented food or beverage is produced.

## 1-1. Plant-protein ingredient

The plant-protein ingredient is not particularly limited as long as it contains a plant protein and serves as a food or beverage material. The specific form of the plant-protein ingredient is not particularly limited as long as it can be a material for fermented food or beverage, and may be any form such as a liquid form, a gel form, or a solid form, but is preferably a liquid form.

The liquid plant-protein ingredient may be any liquid in which at least a plant protein is dissolved and/or dispersed in water, and specific examples include: (i) a liquid obtained by crushing and dispersing food raw materials containing a plant protein in water, and if necessary, removing insoluble matters originating from skins and the like of the food raw materials by any means such as centrifugal filtration, filtration, straining bags, sieves; (ii) a liquid obtained by dispersing in water a dry powder of a food raw material containing a plant protein; (iii) a liquid with an increased content of a plant protein obtained by removing components other than the plant protein from the liquid (i) or (ii) above; and (iv) a liquid obtained by dissolving and/or dispersing a dry powder prepared from any of the liquids (i) to (iii) above in water. Among these, the liquid (ii) above is preferred. Typical examples of these liquid plant-protein ingredients include so-called plant milk.

Examples of the plant protein contained in the plant-protein ingredient include, but are not particularly limited to, plant proteins of the following: legumes such as soybean, pea, lupine bean, broad bean, chickpea, green bean, and kidney bean; grains such as oats, barley, wheat, rye, rice, buckwheat, barnyard millet, foxtail millet, teff, quinoa, and corn; seeds such as canary seed, linseed, almond, cashew nut, hazelnut, pecan nut, macadamia nut, pistachio, walnut, brazil nut, peanut, coconut, hemp, pili nut, chestnut, sesame, and pine nut.

These plant proteins may be used singly or in combination of two or more kinds thereof. Among these plant proteins, preferred is a legume protein, and more preferred is a soybean protein, from the viewpoint of further enhancing the effects of improving various properties related to the plant-protein-fermented food or beverage.

The content of the plant protein contained in the plant-protein ingredient is not particularly limited, and is, for example, 0.5 w/v% or more, 1 w/v% or more, preferably 2 w/v% or more, more preferably 3 w/v% or more, still more preferably 4 w/v% or more, and still more preferably 4.5 w/v% or more. The upper limit of the content range of the plant protein contained in the plant-protein ingredient is not particularly limited, and examples thereof include 30 w/v% or less, 25 w/v% or less, 20 w/v% or less, 15 w/v% or less, 12 w/v% or less, 10 w/v% or less, 8 w/v% or less, 6 w/v% or less, or 5.5 w/v% or less.

In addition to the plant protein, the plant-protein ingredient may contain other raw materials and/or food additives depending on the type of plant-protein-fermented food or beverage (" 1-7. Plant-protein-fermented food or beverage" described later) to be obtained. Examples of other raw materials include a component that is derived from and inevitably coexists with a food raw material containing the above-described plant protein. The food additive is not particularly limited as long as it is a food additive acceptable, and examples thereof include plant fats and oils; seasonings such as salt, sugar, spices, sodium L-glutamate, disodium 5'-ribonucleotide, disodium 5'-inosinate, and disodium 5'-guanylate; antioxidants such as L-ascorbic acid; and fragrances. The timing of adding the other raw materials and/or food additives is not particularly limited, and the other raw materials and/or food additives may be added at the time of being subjected to the fermentation step and/or the step of performing the treatment with an enzyme, or may be added after both the fermentation step and the step of performing the treatment with an enzyme are completed.

## 1-2. Microorganisms used for fermentation

The microorganism used for fermentation of the plant-protein ingredient is not particularly limited as long as it is a microorganism that provides a fermented food or beverage, and examples thereof include lactic acid bacteria, bifidobacteria, koji molds, and yeasts. The lactic acid bacteria are not particularly limited, and examples thereof include lactic acid bacteria belonging to the genus Streptococcus, lactic acid bacteria belonging to the genus Lactobacillus, lactic acid bacteria belonging to the genus Leuconostoc, and lactic acid bacteria belonging to the genus Lactococcus.

These microorganisms may be used singly or in combination of two or more kinds thereof. Among these microorganisms, preferred are lactic acid bacteria, and more preferred are lactic acid bacteria belonging to the genus Streptococcus and lactic acid bacteria belonging to the genus Lactococcus, from the viewpoint of further enhancing the effects of improving various properties related to the plant-protein-fermented food or beverage.

1-3. Plant-cell-wall-derived polysaccharides

The plant-cell-wall-derived polysaccharide used in the present invention is not particularly limited, and examples thereof include pectin, arabinoxylan, hemicellulose, and cellulose.

The origin of pectin is not particularly limited, and examples thereof include citrus peel (for example, lemon, orange, and the like), apple, and beet. The beet from which pectin is derived is Beta vulgaris ssp. vulgaris var. Altissima (a beet also called sugar beet). Further, from the viewpoint of structural characteristics, examples of the pectin include HM pectin having an esterification degree (DE) of 50% or more, LM pectin having a DE of less than 50%, and pectin containing ferulic acid.

These plant-cell-wall-derived polysaccharides may be used singly or in combination of two or more kinds thereof.

Among these plant-cell-wall-derived polysaccharides, pectin is preferable, and beat-derived pectin, HM pectin having a DE of 55% or more, and/or pectin containing ferulic acid (the content of ferulic acid in pectin is, for example, 0.3 to 3 wt%, preferably 0.4 to 2 wt%, and more preferably 0.5 to 1 wt%) is more preferable from the viewpoint of further improving various properties related to a fermented food or beverage using a plant-protein ingredient.

The amount of the plant-cell-wall-derived polysaccharide to be used is not particularly limited, but for example, the amount of the plant-cell-wall-derived polysaccharide per 100 parts by weight of the plant protein is, for example, 0.5 to 100 parts by weight, and from the viewpoint of further improving various properties related to a fermented food or beverage using a plant-protein ingredient, the amount is preferably 1 to 50 parts by weight.

In addition, the amount of the plant-cell-wall-derived polysaccharide to be used is, for example, 0.001 to 0.5 mg as the amount of the plant-cell-wall-derived polysaccharide per 1 U of the multicopper oxidase described later, and is preferably 0.0025 to 0.3 mg, and more preferably 0.005 to 0.2 mg from the viewpoint of further improving various properties related to a fermented food or beverage using a plant-protein ingredient.

1-4. Multicopper oxidase

The multicopper oxidase used in the present invention refers to a group of enzymes containing a plurality of copper atoms in a molecule and oxidizing polyphenol, methoxyphenol, diamine, bilirubin, ascorbic acid, and the like with molecular oxygen. The number of contained copper atoms is usually 2 to 8 as known so far, but this number is not particularly limited because it varies depending on the state of the enzyme preparation at the time of analysis and the analysis method. Examples of the enzyme classified as the multicopper oxidase include laccase, bilirubin oxidase, ascorbic acid oxidase, and cellulose plasmin.

These multicopper oxidases may be used singly or in combination of two or more kinds thereof. Among these multicopper oxidases, laccase is preferable from the viewpoint of further improving various properties related to a fermented food or beverage using a plant-protein ingredient.

Laccase is an enzyme having phenol oxidase activity (EC 1.10.3.2). Specific examples of the laccase include laccases derived from microorganisms such as fungi and bacteria, and more specific examples thereof include laccases derived from the genus Aspergillus, the genus Neurospora, the genus Podospora, the genus Botrytis, the genus Collybia, the genus Fomes, the genus Lentinus, the genus Pleurotus, the genus Pycnoporus, the genus Pyricularia, the genus Trametes, the genus Rhizoctonia, the genus Rigidoporus, the genus Coprinus, the genus Psatyrella, the genus Myceliophtera, the genus Schtalidium, the genus Polyporus, the genus Phlebia, the genus Coriolus.

These laccases may be used singly or in combination of two or more kinds thereof. Among these laccases, laccase derived from the genus Trametes and laccase derived from the genus Aspergillus (more preferably laccase derived from Aspergillus oryzae) are preferred, and laccase derived from the genus Trametes is further preferred, from the viewpoint of further improving various properties related to a fermented food or beverage using a plant-protein ingredient.

The amount of the multicopper oxidase to be used is not particularly limited, but for example, the amount of the multicopper oxidase per 1 g of the plant protein is, for example, 500 to 50,000 U, and from the viewpoint of further improving various properties related to a fermented food or beverage using a plant-protein ingredient, the amount is preferably 1,500 to 25,000 U.

For the activity of multicopper oxidase, when an enzyme liquid is added to 2,2'-Azino-di-[3-ethylbenzthiazoline sulfonate] (ABTS) as a substrate, the amount of enzyme that catalyzes the oxidation of 1 $\mu$mol of ABTS per minute is defined as 1 unit (U).

1-5. Steps order

The step of: fermenting a plant-protein ingredient and the step of performing a treatment with a plant-cell-wall-derived polysaccharide and a multicopper oxidase are performed in an arbitrary order. That is, either one of the fermentation step and the treatment step may be performed first, and after completion of the one step, the other step may be performed, or both steps may be performed simultaneously. Furthermore, in a case where both steps are performed at the same time, the start timings of both steps may be the same time, or the start timing of one of the steps may be advanced.

From the viewpoint of further enhancing the effects of improving various properties related to the plant-protein-fermented food or beverage, it is preferable that the fermentation step and the treatment step may be performed simultaneously. It is more preferable that the both steps are performed simultaneously, with the start timing of one of the steps being advanced. It is further more preferable that the fermentation step is started first, and then, both of the fermentation step and the treatment step are performed in parallel.

1-6. Reaction conditions, etc.

The reaction conditions in the step of fermenting the plant-protein ingredient can be appropriately selected by those skilled in the art in consideration of the thermal stability of microorganisms, whether the treatment step is simultaneously performed, and the like, and the reaction conditions are, for example, 20 to 40°C, preferably 25 to 30°C. The time for the fermentation step can be appropriately determined by those skilled in the art according to the type of the plant-protein-fermented food or beverage to be obtained, and is, for example, 8 to 50 hours, preferably 15 to 40 hours, and more preferably 20 to 30 hours. The fermentation step can be advanced, for example by 3 to 15 hours, preferably by 5 to 10 hours, when its start is advanced from the start of the treatment step. In the fermentation step performed in advance to the start of the treatment step, a plant-protein ingredient can be appropriately added along with the growth of microorganisms.

The reaction conditions in the step of performing the treatment with a plant-cell-wall-derived polysaccharide and a multicopper oxidase can be appropriately determined by those skilled in the art in consideration of the optimal temperature for the multicopper oxidase, whether the fermentation step is simultaneously performed, and the like, and the reaction conditions are, for example, 4 to 80°C, preferably 15 to 50°C, more preferably 20 to 40°C, and furthermore preferably 25 to 30°C. The time for the treatment step can be appropriately determined by those skilled in the art according to the levels of the various properties to be achieved, and is, for example, 1 to 30 hours, preferably 8 to 25 hours, and more preferably 15 to 20 hours.

1-7. Plant-protein-fermented food or beverage

The plant-protein-fermented food or beverage produced by the production method of the present invention is not particularly limited. Examples of the form of the plant-protein-fermented food or beverage include a solid form, a gel form, and a liquid form. Specific examples of the plant-protein-fermented food or beverage include a plant yogurt (an example of a gel-like food), a plant yogurt beverage (an example of a beverage), a plant yogurt paste (an example of a paste-like food. It is also used as a food material), and plant cheese (an example of a solid food).

From the viewpoint of further enhancing the effects of improving various properties related to the plant-protein-fermented food or beverage, the preferable form of the plant-protein-fermented food or beverage is the gel form, and the preferable specific example of the plant-protein-fermented food or beverage is plant yogurt.

2. Modifier for plant-protein-fermented food or beverage

Using a combination of a plant-cell-wall-derived polysaccharide and a multicopper oxidase for the production of the plant-protein-fermented food or beverage enables such modification as to improve various properties such as stress, water retention properties, syneresis-suppressing properties, or digestive properties of the fermented food or beverage itself. The present invention, therefore, also provides a modifier for a plant-protein-fermented food or beverage, the modifier containing a plant-cell-wall-derived polysaccharide and a multicopper oxidase.

More specific examples of the modifier for the plant-protein-fermented food or beverage include those used as a stress improver for the plant-protein-fermented food or beverage, a water retention property improver for the plant-protein-fermented food or beverage, a syneresis inhibitor for the plant-protein-fermented food or beverage, and/or a digestive property improver for the plant-protein-fermented food or beverage.

The types and amounts of components used in the modifier for the plant-protein-fermented food or beverage are as shown in the description in the section of "1. Method for producing plant-protein-fermented food or beverage" shown above.

3. Agent for shortening fermentation time in production of plant-protein-fermented food or beverage

Using a combination of a plant-cell-wall-derived polysaccharide and a multicopper oxidase for the production of the plant-protein-fermented food or beverage enables to shorten the fermentation time during production (that is, enables fast brewing). The present invention, therefore, also provides an agent for shortening the fermentation time in the production of a plant-protein-fermented food or beverage (that is, a fast brewing agent in the production of a plant-protein-fermented food or beverage), the agent containing a plant-cell-wall-derived polysaccharide and a multicopper oxidase.

The types and amounts of components used in the agent for shortening the fermentation time in the production of a plant-protein-fermented food or beverage are as shown in the description in the section of "1. Method for producing plant-protein-fermented food or beverage" shown above.

EXAMPLES

Hereinafter, the present invention will be described specifically with reference to examples, but the present invention is not to be interpreted as being limited to the following examples.

[Test Example 1]

(1) Materials used

The materials shown in Table 1 below were used.

[Table 1]

| Plant-protein ingredient | Soy milk | Content of plant protein 5 w/v% | "Organic Soymilk, also for Tofu-making" (Sujatha Meiraku Group) |
|---|---|---|---|
| Microorganism used for fermentation | Lactic acid bacteria | Lactococcus lactis subsp. cremoris strain BRF | "King's Yogurt Seed Bacteria" (OHTA'S ISAN Co., Ltd.) |
| | Lactic acid bacteria | Streptococcus thermophilus strain KI-1 | |
| Plant-cell-wall-derived polysaccharide | Pectin | Derived from sugar beet DE55% or more | GENU pectin type BETA BI-J (Sansho Co., Ltd.) |
| Multicopper oxidase | Laccase | Derived from genus Trametes | Laccase Y120 (Amano Enzyme Co., Ltd.) |

(2) Method for measuring laccase activity value

The enzyme activity of laccase was measured by the method described below using 2,2'-Azino-di-[3-ethylbenzthiazoline sulfonate] (ABTS, manufactured by Boehringer Mannheim) as a substrate.

ABTS was dissolved in 25 mM citrate buffer (pH 3.2) at a concentration of 1.0 mg/ml to prepare a substrate solution. After 3.0 ml of this substrate solution was placed in a cuvette and preheated at 25°C, then 0.1 ml of the enzyme liquid was added thereto, and the mixture was stirred and incubated at 25°C. The absorbance thereof at 405 nm after 1 minute and 3 minutes was measured. The amount of the enzyme that increased the absorbance at 405 nm by 1.0 OD per minute under this condition was defined as 1 unit (U).

(3) Procedure

Lactic acid bacteria (inoculum) were inoculated into 5 mL of soy milk, and the inoculum was pre-precultured at 28°C for 4 hours. The culture thus obtained, 0.1 mL, was inoculated into 5 mL of fresh soy milk, and was precultured at 28°C for 4 hours. The culture thus obtained, 1 mL, was added to 50 mL of fresh soy milk and mixed, and pectin and/or laccase were further added in the amounts shown in Tables 2A and 2B and mixed. The mixture was left standing at 28°C for 16 hours, and then left standing in a refrigerator for 2 to 4 hours (only when subjected to the test of syneresis-suppressing properties, the standing conditions were the conditions shown in the section (4-3) below) to obtain soy milk yogurt.

(4) Tests of various properties

The obtained soy milk yogurt was tested for the following various properties. The results are shown in Tables 2A and 2B.

(4-1) Stress

The stress of the obtained soy milk yogurt was measured using a rheometer (manufactured by Sun Scientific Co., Ltd.). The relative stress (%) when the stress of the soy milk yogurt of Comparative Example 1 was taken as 100% was derived. It can be evaluated that the stress is improved as the value of the relative stress is larger.

(4-2) Water retention properties

The weight of the obtained soy milk yogurt was measured. Then the soy milk yogurt was centrifuged under the conditions of 1000 × g, 10 minutes, and 20°C, the supernatant was collected, and the weight of the remaining soy milk yogurt was measured. The water retention power (%) was derived based on the following equation. It can be evaluated that the water retention power is improved as the value of the water retention power is larger.

[Mathematical formula 1]

$$\text{Water retention power (\%)} = 100 - \{(W1 - W2) / W1 \times 100\}$$

W1: Weight (g) before centrifugation
W2: Weight (g) obtained by removing supernatant after centrifugation

(4-3) Syneresis-suppressing properties

The weight (V1 (g)) of soy milk yogurt was measured before refrigerated storage. The whole amount of the measured soy milk yogurt was placed on gauze covering a liquid receiving container, and stored in a refrigerator for 24 hours. After 24 hours, the weight of the naturally separated liquid accumulated in the liquid receiving container was measured. The weight (V2 (g)) of soy milk yogurt after 24 hours of refrigerated storage was calculated by subtracting the weight of the liquid from V1 (g). The syneresis properties (%) was derived based on the following equation. It can be evaluated that the syneresis-suppressing properties are improved as the value of the syneresis properties is smaller.

[Mathematical formula 2]

$$\text{Syneresis properties (\%)} = 100 - (V2 / V1 \times 100)$$

V1: Weight (g) of soy milk yogurt before refrigeration
V2: weight (g) of soy milk yogurt after 24 hours of refrigerated storage

[Table 2A]

| | | | Example | | | |
|---|---|---|---|---|---|---|
| | | | 1 | 2 | 3 | 4 |
| Added amount | Pectin | Per 100 parts by weight of soy protein (parts by weight) | 1.6 | 3.2 | 8 | 16 |
| | | Per 1 U of laccase (mg) | 0.0074 | 0.0074 | 0.0074 | 0.0074 |
| | Laccase | Per 1 g of soy protein (U) | 2160 | 4320 | 10800 | 21600 |
| Relative stress (%) | | | 121 | 136 | 150 | 171 |
| Water retention power (%) | | | 90.2 | 92.6 | 95.1 | 96.5 |
| Syneresis properties (%) | | | 12.2 | 8.1 | 5.8 | 4.6 |

[Table 2B]

| | | | Comparative Example | | | | | |
|---|---|---|---|---|---|---|---|---|
| | | | 1 | 2 | 3 | 4 | 5 | 6 |
| Added amount | Pectin | Per 100 parts by weight of soy protein (parts by weight) | 0 | 0 | 0 | 0 | 0 | 16 |
| | | Per 1 U of laccase (mg) | | | | | | |
| | Laccase | Per 1 g of soy protein (U) | 0 | 2160 | 4320 | 10800 | 21600 | 0 |
| Relative stress (%) | | | 100 | 107 | 121 | 129 | 129 | 100 |
| Water retention power (%) | | | 88.5 | 89.6 | 90.6 | 91.9 | 91.8 | 86.2 |
| Syneresis properties (%) | | | 18.5 | 15.6 | 13.2 | 10.5 | 9.4 | 16.6 |

As shown in Table 2B, each of pectin and laccase alone has no or insufficient function of improving various properties of soy milk yogurt, but as shown in Table 2A, by combining pectin and laccase, various properties of soy milk yogurt could be remarkably improved as compared with the case of using the same amount of enzyme alone.

[Test Example 2]

Each soybean milk yogurt was prepared in the same manner as in Test Example 1 except that pectin and/or laccase were used in the amounts shown in Table 3, and the relative stress was derived in the same manner as in (4-1) in Test Example 1. The results are shown in Table 3.

[Table 3]

| | | | Example | Comparative Example | |
|---|---|---|---|---|---|
| | | | 5 | 7 | 8 |
| Added amount | Pectin | Per 100 parts by weight of soy protein (parts by weight) | 40 | 0 | 0 |
| | | Per 1 U of laccase (mg) | 0.185 | | |
| | Laccase | Per 1 g of soy protein (U) | 2160 | 0 | 2160 |
| Relative stress (%) | | | 257 | 100 | 107 |

As shown in Table 3, when laccase and pectin were used in combination, the effect of increasing the stress by 2.4 times as compared with the case of using laccase alone was observed.

[Test Example 3]

Each soybean milk yogurt was prepared in the same manner as in Test Example 1 except that pectin and/or laccase were used in the amounts shown in Table 4. During the preparation, the pH was measured over time from the time point when pectin and/or laccase was added, and the time until the pH reached 5 was measured. It can be evaluated that the shorter the time is, the higher the effect of shortening the fermentation time is. The results are shown in Table 4. In addition, the amount of lactic acid was measured using Lactate Assay Kit-WST (DOJINDO LABORATORIES) over time from the time point when pectin and/or laccase was added. The amount of lactic acid after 36 hours when a sufficient time had elapsed after completion of the soy milk yogurt is shown in Table 4.

[Table 4]

| | | | Example | Comparative Example | | |
|---|---|---|---|---|---|---|
| | | | 6 | 9 | 10 | 11 |
| Added amount | Pectin | Per 100 parts by weight of soy protein (parts by weight) | 16 | 0 | 0 | 16 |
| | | Per 1 U of laccase (mg) | 0.007 | 0 | 0 | / |
| | Laccase | Per 1 g of soy protein (U) | 21600 | 0 | 21600 | 0 |
| Time until pH reached 5 (hours) | | | 13 | 22 | 18 | 22 |
| Amount of lactic acid after 36 hours (mM) | | | 95 | 93 | 93 | Unmeasured |

As shown in Table 4, the fermentation time could be remarkably shortened by using pectin and laccase for the production of soy milk yogurt. In general, lactic acid is released as fermentation proceeds, and when the amount of lactic acid is excessive, the taste is deteriorated. However, even when fermentation was accelerated by the use of pectin and laccase, the amount of lactic acid was equivalent to that in Comparative Example as shown in Table 4, and no adverse effect on taste was observed.

[Test Example 4]

The following digestion test was performed on the soy milk yogurts of Example 4, Comparative Example 1, and Comparative Example 5. Each soy milk yogurt, 5 mL, and a simulated gastric fluid (77 mL of Purified Water, 13 mL of McIlvain Buffer pH 5.0, 4.39 g of NaCl, 0.22 g ofKCl, 0.04 g of CaCl$_2$, final concentration 0.0065% pepsin) were mixed, and the mixture was reacted at 37°C and 60 rpm for 80 minutes. At that time, 1N HCl was added every 10 minutes to adjust the pH to 3.0. After completion of the reaction, boiling and cooling were performed, and the free amino nitrogen content after digestion was measured. It can be evaluated that the higher the free amino nitrogen content is, the better the digestive properties are. The results are shown in Fig. 1.

As shown in Test Example 1, the soy milk yogurt of Example 4 had a remarkable effect of increasing the stress as compared with Comparative Example 1 and Comparative Example 5, but unexpectedly, it was confirmed that the digestive properties thereof were rather improved as compared with Comparative Example 1 and Comparative Example 5 as shown in Fig. 1.

**Claims**

1. A method for producing a plant-protein-fermented food or beverage, comprising the steps of:

   fermenting a plant-protein ingredient; and
   performing a treatment with a plant-cell-wall-derived polysaccharide and a multicopper oxidase.

2. The method according to claim 1, wherein the plant-cell-wall-derived polysaccharide is pectin.

3. The method according to claim 2, wherein the pectin is derived from beet.

4. The method according to claim 1, wherein the multicopper oxidase is laccase.

**5.** The method according to claim 1, wherein the plant-protein ingredient is plant milk.

**6.** The method according to claim 1, wherein the plant-protein-fermented food or beverage is plant yogurt.

**7.** A modifier for a plant-protein-fermented food or beverage, the modifier comprising a plant-cell-wall-derived polysaccharide and a multicopper oxidase.

**8.** The modifier according to claim 7, used as a stress improver for a plant-protein-fermented food or beverage.

**9.** The modifier according to claim 7, used as a water retention property improver for a plant-protein-fermented food or beverage.

**10.** The modifier according to claim 7, used as a syneresis inhibitor for a plant-protein-fermented food or beverage.

**11.** The modifier according to claim 7, used as a digestive property improver for a plant-protein-fermented food or beverage.

**12.** An agent for shortening fermentation time in production of a plant-protein-fermented food or beverage, the agent comprising a plant-cell-wall-derived polysaccharide and a multicopper oxidase.

FIG. 1

## INTERNATIONAL SEARCH REPORT

| International application No. |
|---|
| **PCT/JP2022/031885** |

**A. CLASSIFICATION OF SUBJECT MATTER**

*A23L 5/00*(2016.01)i; *A23J 3/14*(2006.01)i; *A23L 11/50*(2021.01)i; *C12N 9/04*(2006.01)i
FI:  A23L5/00 J; A23L11/50; A23L5/00 D; A23J3/14; C12N9/04

According to International Patent Classification (IPC) or to both national classification and IPC

**B. FIELDS SEARCHED**

Minimum documentation searched (classification system followed by classification symbols)

A23L5/00; A23J3/14; A23L11/50; C12N9/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | 食物アレルギー体験レポーター岡夫婦 [オンライン], 16 March 2021 [retrieval date 17 October 2022], Internet:<URL:https://oka-allergy.com/2021/03/16/almond-milk-yogurt/> almond milk yogurt, non-official translation (Food allergy experience reporter Mr. and Mrs. OKA [online]) | 1-12 |
| A | 館長の台所 [オンライン], 10 February 2016 [retrieval date 17 October 2022], Internet:<URL:https://k-daidokoro.com/archives/7188> coconut milk yogurt, non-official translation (Kancho no Daidokoro [online]) | 1-12 |
| A | JP 2006-524047 A (RAISIO BENECOL OY) 26 October 2006 (2006-10-26) | 1-12 |
| A | JP 44-014772 B1 (MOCHIZUKI, Teijiro) 01 July 1969 (1969-07-01) | 1-12 |
| A | STRUCH, Marlene et al. Laccase-catalysed cross-linking of a yoghurt-like model system made from skimmed milk with added food-grade mediators. International Dairy Journal. 2015, vol. 49, pp. 89-94 abstract | 1-12 |
| A | JP 11-276162 A (AMANO PHARMACEUT CO LTD) 12 October 1999 (1999-10-12) | 1-12 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 October 2022** | **15 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/031885**

### C.    DOCUMENTS CONSIDERED TO BE RELEVANT

| Category* | Citation of document, with indication, where appropriate, of the relevant passages | Relevant to claim No. |
|---|---|---|
| P, A | WO 2022/173018 A1 (AMANO ENZYME INC) 18 August 2022 (2022-08-18) | 1-12 |
| P, A | WO 2022/054880 A1 (AMANO ENZYME INC) 17 March 2022 (2022-03-17) | 1-12 |

Form PCT/ISA/210 (second sheet) (January 2015)

INTERNATIONAL SEARCH REPORT
Information on patent family members

International application No.

**PCT/JP2022/031885**

| Patent document cited in search report | | | Publication date (day/month/year) | Patent family member(s) | | | Publication date (day/month/year) |
|---|---|---|---|---|---|---|---|
| JP | 2006-524047 | A | 26 October 2006 | US | 2007/0026119 | A1 | |
| | | | | WO | 2004/093571 | A1 | |
| | | | | EP | 1615511 | A1 | |
| JP | 44-014772 | B1 | 01 July 1969 | (Family: none) | | | |
| JP | 11-276162 | A | 12 October 1999 | US | 6121013 | A | |
| | | | | US | 2002/0009770 | A1 | |
| | | | | EP | 947142 | A2 | |
| WO | 2022/173018 | A1 | 18 August 2022 | (Family: none) | | | |
| WO | 2022/054880 | A1 | 17 March 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11276162 A
- JP 2009273412 A
- JP H06197688 A
- WO 2012121090 A
- JP H06276933 A